# EUROPEAN PATENT APPLICATION

(11) **EP 2 325 172 A1**
(43) Date of publication of application: **25.05.2011**
(21) Application number: 09384005.6
(22) Date of filing: 02.11.2009
(51) Int. Cl.: C07D 231/12, A61K 31/415, A61P 29/00

(54) **Co-crystals of celecoxib and L-proline**

(71) Applicant: Laboratorios del. Dr. Esteve, S.A., 08041 Barcelona (ES)
(72) Inventor: Plata Salaman, Carlos Ramon, 08950 Esplugues de Llobregat (Barcelona) (ES); Tesson, Nicolas, 08902 L`Hospitalet de Llobregat (ES)
(74) Representative: Peters, Hajo

(57) **Abstract**

The present invention relates to co-crystals of celecoxib and L-proline, processes for preparation of the same and their use as medicament or in pharmaceutical compositions, more particularly for the treatment of pain.

## Description

The present invention relates to co-crystals of celecoxib and L-proline, processes for preparation of the same and their use as medicament or in pharmaceutical compositions, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis (such as osteoarthritis or rheumatoid arthritis), or pain of unknown origin, as fibromyalgia. Acute pain usually follows non-neural tissue injury, for example tissue damage from surgery or inflammation, or migraine.

There are many drugs (analgesics) that are known to be useful in the treatment or management of pain. In principle, these analgesics are classified by their mechanism of action into opioids and non-opioids analgesics.

Opioids, also known as derivatives of morphine, are frequently used as analgesics in pain. In general, derivatives of morphine are indicated for the treatment of moderate to acute pain in human. The analgesic effect is obtained through their action on morphinic (opioid) receptors, preferably µ-receptors. Among these derivatives of morphine, may be mentioned morphine, codeine, pethidine, dextropropoxyphene methadone, and others. However, opioids exhibit severe drawbacks, such as tolerance, dependence, risk of addiction and abuse. Therefore, opioids cannot always be given repeatedly or at higher doses as analgesics. For reviewing side effects of opioids one can mention J. Jaffe in "Goodman and Gilman's, The Pharmacological Basis of Therapeutics", 8th edition; Gilman et al.; Pergamon Press, New York, 1990, Chapter 22, pages 522-573.

One commonly known group of non-opioid analgesic agents are the well established COX-inhibitors. COX-inhibitors, as their name implies, are effective in relieving pain via inhibiting the enzymes cyclooxygenase (COX) I (constitutive) and/or II (inducible) involved in the production of prostaglandins. COX-II is the isoform of the enzyme that has been shown to be induced by pro-inflammatory stimuli and has been postulated to be primarily responsible for the synthesis of prostanoid mediators of pain, inflammation, and fever. COX-II is also involved in ovulation, implantation and closure of the ductus arteriosus, regulation of renal function, and central nervous system functions (fever induction, pain perception and cognitive function). It may also play a role in ulcer healing. COX-II has been identified in tissue around gastric ulcers in man but its relevance to ulcer healing has not been established.

COX-inhibitors include non-steroidal anti-inflammatory drugs (NSAIDs) with acetylsalicylic acid known under its trademark Aspirin - despite being more than 100 years old - being an outstandingly used pharmaceutical. Besides Aspirin other COX-inhibitors whose use generally is also centred on anti-inflammatory action like ibuprofen, naproxen or diclofenac are among the worldwide most frequently applied pharmaceutical compounds. One COX-II inhibitor is celecoxib, an anti-inflammatory and pain killer drug and one of the most used drugs for the treatment for chronic musculo-skeletal inflammatory illnesses. In addition, it has been recently shown that paracetamol also functions as a COX-II inhibitor (FASEB J., 2008 Feb; 22(2):383-90).

Celecoxib, whose chemical name is 4-[5-(4-methylphenyl)-3-(trifluoromethyl)pyrazol-1-yl]benzenesulfonamide, has the following formula:

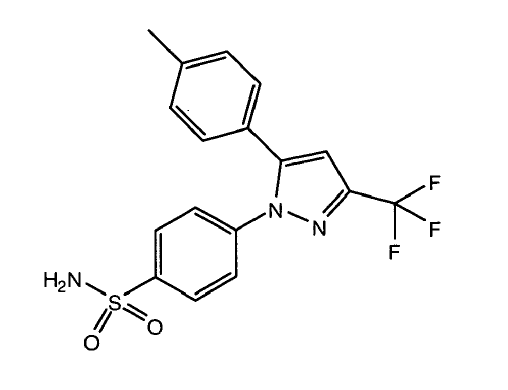

Celecoxib is an oral, highly selective COX-II inhibitor, which is indicated for the treatment of symptomatic relief in the treatment of osteoarthritis, rheumatoid arthritis and ankylosing spondylitis (Goldenberg MM. Celecoxib, a selective cyclooxygenase-2 inhibitor for the treatment of rheumatoid arthritis and osteoarthritis. Clin. Ther. 1999, 21, 1497-1513; http://www.medsafe.govt.nz/profs/Datasheet/c/Cetebrexcap.htm). In addition, celecoxib is also known to be used for the treatment of sciatica, frozen shoulder, dysmenorrhea and familial adenomatons polyposis. The high selectivity towards COX-II allows celecoxib and other COX-II inhibitors to reduce inflammation (and pain) while minimizing gastrointestinal adverse drug reactions (e.g. stomach ulcers) that are common with non-selective NSAIDs.

The advantage of COX-inhibitors in general, i.e. COX-I and COX-II inhibitors, is that they do not produce tolerance or physical dependence and are also not associated with abuse or addiction. However, for a number of COX-inhibitors a low solubility in water exists. This is especially true for the very popular and widely used and distributed members of the group of COX-inhibitors, naproxen, diclofenac and ibuprofen, whose poor solubility is a published fact, that has lead to considerably efforts for improvement by using solution enhancers etc. in their formulation.

The COX-II inhibitor celecoxib is also well-known to be only slightly soluble limiting inter alia its use in pharmaceutical formulations and its bioavailability. For instance, the marketed formulation Celebrex®, that is administered orally, contains celecoxib in its crystalline form and has an absolute bioavailability ranging from about 20-40% in dogs attributed to a reduced absorption (Susan K. Paulson, Margaret B. Vaughn, Susan M. Jessen, Yvette Lawal, Christopher J. Gresk, Bo Yan, Timothy J. Maziasz, Chyung S. Cook. Aziz Karim, 2001, "Pharmacokinetics of Celecoxib after Oral Administration in Dogs and Humans: Effect of Food Site of Absorption", J. Pharmacol.& Experim. Therapeutics, 297(2), 638-645). This poor bioavailability limits the distribution and target organ delivery of celecoxib, and hence, the efficacy of this drug.

Therefore, there is a need to improve celecoxib oral absorption profile and to reduce the dose required to achieve therapeutic activity that moreover could minimize adverse effects and drug interactions.

However, celecoxib is weakly acidic with a pKa in water of 11.1 (http://www.medsafe.govt.nz/profs/Datasheet/c/Celebrexcap.htm) leading to the nonionized form at all physiological pH ranges. Therefore, celecoxib is a high permeable drug exhibiting a very low water solubility of 7µg/ml (Neelam Seedher, Somm Bhatia, 2003 "Solubility enhancement of Cox-2 Inhibitors Using various Solvent Systems", AAPS Pharm. Sci. Tech., 4(3), 1-9). These physico-chemical properties make celecoxib to be considered as class II in the Biopharmaceutical Classification System (BCS) (Mehran Yazdanian, Katherine Briggs, Corinne Jankovskty, Amale Hawi, 2004, "The "High solubility" Definition of the Current FDA Guidance on Biopharmaceutical Classification System May Be Too Strict for Acidic Drugs", Pharm. Res., 2004, 21(2), 293-9). Consequently the extent of celecoxib oral absorption appears to be limited by its bad solubility and slow dissolution rate, which leads to an inadequate dissolution in gastrointestinal fluids (Susan K. Paulson, Margaret B. Vaughn, Susan M. Jessen, Yvette Lawal, Christopher J. Gresk, Bo Yan, Timothy J. Maziasz, Chyung S. Cook. Aziz Karim, "Pharmacokinetics of Celecoxib after Oral Administration in Dogs and Humans: Effect of Food Site of Absorption", J. Pharmacol.& Experim Therapeutics, 2001, 297(2), 638-645).

In order to increase absorption rate and extent and hence bioavailability of celecoxib, it is an object of the present invention to improve the aqueous solubility and dissolution rate of celecoxib as well as the retard of precipitation of dissolved celecoxib by providing new drugable forms of celecoxib.

It is a further object of the present invention to provide a new drugable form of celecoxib which should combine more than one, preferably most of the following improvements and advantages, respectively:
● improvement of physicochemical properties in order to facilitate the formulation, the manufacture, or to enhance the absorption and/or the bioavailability: thus
● being more active when compared to celecoxib alone;
● providing a form of celecoxib having a beneficial pharmacological effect in itself, thus allowing for a highly efficient dose/weight relation of the final active principle; or even
● allowing the use of a lower therapeutic dose of celecoxib;
● being easily obtainable, easy to manufacture;
● allowing more flexibility in formulating, or facilitating its formulation;
● being highly soluble, thus allowing better dissolution rates, especially if dissolving in an aqueous physiological surrounding;
● improving stability of the celecoxib;
● allowing new routes of administration.

It was found, surprisingly, that celecoxib and L-proline as starting elements are suitable to form co-crystals. These co-crystals exhibit improved properties if compared to celecoxib alone and/or to a mixture of celecoxib and L-proline.

Therefore, in a first aspect thereof, the object of the present invention is solved by providing a new co-crystal of celecoxib either as a free base or as a physiologically acceptable salt and L-proline either as a free base or as a physiologically acceptable salt. These co-crystals show improved properties if compared to celecoxib alone and/or to a mixture of celecoxib and L-proline. The co-crystals thus obtained have a specific stoichiometry which depends upon the structure of L-proline as co-crystal former. Under the proper circumstance this is also another advantage of these new solid drugable forms possibly achieving some modulation of the pharmacological effects. While APIs (Active Pharmaceutical Ingredients) like celecoxib in general have been recognized to form crystalline polymorphs, solvates, hydrates and amorphous forms for a number of years, there is little knowledge about which APls will form co-crystals. Co-crystals are a specific type of crystalline form which provide a new avenue to modulate the API form and thus to modulate API properties. Co-crystals contain an API and at least one other component which crystallize together. Selection of the other component helps to determine whether a co-crystal will form and what properties the co-crystal will have. Just as a polymorph, solvate, hydrate or amorphous form of an API can modulate stability, solubility, and hygroscopicity, a co-crystal can modulate those same properties.

The physico-chemical properties of the co-crystal celecoxib and L-proline are improved. The formulation of the association as a co-crystal is even easier with a solid to manipulate and enhance stability. The solubility is also enhanced. Another advantage is that the combination of one active principle like celecoxib and a co-crystal former (L-proline) into one unique species allows for a better Pharmacokinetic/Pharmacodynamic (PKPD) which helps in the treatment of pain.

"Drugable form (of celecoxib)" as used herein is defined as any form (salt, amorphous, crystal, solution, dispersion, mixture etc,) that celecoxib might take which still can be formulated into a pharmaceutical formulation usable as a medicament to treat a disease or a symptom, especially pain.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction which is neither ionic nor covalent and includes for example: hydrogen bonds, van der Waals forces, and interactions. Solvates of celecoxib that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In scientific literature there currently is some discussion on the proper use of the word co-crystal (see for example Desiraju, Cryst. Eng. Comm., 2003, 5(82), 466-467 and Dunitz, Cryst. Eng. Comm., 2003, 5(91), 506). A recent article by Zaworotko (M. J. Zaworotko, Crystal Growth & Design, Vol. 7, No. 1, 2007, 4-9) gives a definition of co-crystal which is in line with the definition given above and thus also is a definition of "co-crystal" according to this invention. According to this article *"a co-crystal is a multiple component crystal in which all components are solid under ambient conditions when in their pure form. These components consist of a target molecule or ion and a molecular co-crystal former(s); when in a co-crystal, they coexist at the molecular level within a single crystal."*

"Co-crystal former" as used herein is defined as L-proline with which celecoxib is able to form co-crystals.

"Active agents" are APIs which show a pharmaceutical effect and thus can be identified as being pharmaceutically active. In a more narrow sense this definition is encompassing all APIs being marketed or under clinical trial for the treatment of diseases. "Active agents with analgesic activity" are APIs (Active Pharmaceutical Ingredients) which show efficacy in well-known animal models of pain and thus can be identified as being analgesics. In a more narrow sense this definition is encompassing all APIs being marketed or under clinical trial for a labelling including an indication falling under the definition of pain, including also migraine. These indications might include acute pain, chronic pain, neuropathic pain, hyperalgesia, allodynia or cancer pain, including diabetic neuropathy or diabetic peripheral neuropathy, osteoarthritis or fibromyalgia and all their subforms. An example of an "active agents with analgesic activity" is paracetamol.

The term "salt" is to be understood as meaning any form of celecoxib or L-proline according to the invention in which this assumes an ionic form or is charged and is coupled with a counter-ion (a cation or anion) or is in solution. By this are also to be understood complexes of celecoxib or L-proline with other molecules and ions, in particular complexes which are complexed via ionic interactions. This also includes physiologically acceptable salt.

The term "solvate" according to this invention is to be understood as meaning any form of the tramadol or paracetamol in which the compound has attached to it via non-covalent binding a solvent (most likely a polar solvent) especially including hydrates and alcoholates, e.g. mono-hydrate.

In one embodiment of the co-crystal according to the present invention, the ratio between celecoxib and L-proline is chosen in such a way that if compared to either celecoxib alone and/or to a mixture of celecoxib and L-proline
● the solubility of the co-crystal is increased; and/or
● the dose response of the co-crystal is increased; and/or
● the efficacy of the co-crystal is increased; and/or
● the dissolution of the co-crystal is increased; and/or
● the bioavailability of the co-crystal is increased; and/or
● the stability of the co-crystal is increased; and/or
● the hygroscopicity of the co-crystal is decreased; and/or
● the form diversity of the co-crystal is decreased; and/or
● the morphology of the co-crystal is modulated.

"Mixture of celecoxib and L-proline" is defined as a mixture of the celecoxib with L-proline which is only a physical mixture without any coupling forces between the compounds and thus neither includes salts nor co-crystals.

In a preferred embodiment of the co-crystal according to the present invention the molecular ratio between celecoxib and L-proline is 1:2.

In a preferred embodiment of the co-crystal according to the present invention, the endothermic sharp peak corresponding to the melting point has an onset at 201.4°C.

In a preferred embodiment the co-crystal according to the present invention shows a X-Ray powder diffraction pattern with peaks [20] at 5.29, 5.77, 7.28, 9.71, 10.60, 11.57, 12.39, 13.05, 13.56, 14.46, 15.11, 15.45, 15.83, 16.14, 16.96, 17.25, 17.67, 17.92, 18.08, 18.70, 19.37, 19.64, 19.85, 20.17, 20.43, 20.79, 21.17, 21.58, 21.85, 22.15, 22.30, 22.74, 23.12, 23.78, 24.04, 24.72, 24.87, 25.43, 26.57, 26.97, 27.66, 28.04, 28.37, 28.59, 29.11, 29.56, 29.78, 30.41, 30.60, 31.56, 32.21, 32.74, 33.13, 33.35, 33.75, 34.97, 35.80, 36.87, 37.48, 37.69, 38.08 and 38.87. In this respect the 2θ values were obtained using copper radiation (Cu_{kα1} 1.54060A).

In a preferred embodiment the co-crystal according to the present invention has an orthorhombic unit cell with the following dimensions:
a = 9.61 Å,
b = 20.07 Å, and
c = 30.63 Å.

In a preferred embodiment the co-crystal according to the present invention shows a Fourier Transform Infra Red spectrum with absorption bands at 3283.4 (m), 3079.5 (m), 2985.0 (m), 2680.0 (w), 1599.0 (m), 1500.1 (m), 1469.8 (s), 1449.2 (m), 1373.7 (s), 1345.4 (s), 1331.6 (s), 1267.3 (m), 1231.8 (s), 1172.0 (s), 1137.1 (s), 1111.9 (m), 1093.9 (m), 975.5 (m), 841.3 (s), 817.0 (s), 760.5 (m), 684.0 (m), 627.0 (s), 616.4 (s) 557.3 (m), 544.5 (m), 479.1 (m) cm⁻¹.

Another aspect of the present invention relates to a process for the production of a co-crystal according to the present invention comprising the steps of:
- (a): dissolving or suspending celecoxib and L-proline in a solvent or mixture of solvents,
- (b): optionally mixing or stirring the solution or suspension,
- (c): optionally before, during or after step (b) cooling or keeping the mixed solution/suspension to or at ambient temperature or below;
- (d): optionally filtering-off and/or washing the resulting solid with a solvent, and
- (e): drying the solid optionally at ambient temperature and/or vacuum.

"Ambient temperature" is defined here as a temperature between 20 and 25°C, preferably being 20°C. Preferably the cooling in step (c) is done from ambient temperature to approximately 0-5°C.

The solvents usable in this process include water or organic solvents, preferably solvents selected from dichloromethane, methanol, acetone, isobutyl acetate, acetonitrile, ethyl acetate, 2-butanol, dimethylcarbonate, chlorobenzene, butylether, diisopropylether, dimethylformamide, ethanol, hexane, isopropanol, methyl ethyl ketone, methyl isobutyl ketone, methyl t-butyl ether, 3-pentanone, toluene and 1,1,1-trichloroethane, most preferably selected from dichloromethane, toluene, isobutyl acetate and methanol.

In one embodiment of the process for the production of a pharmaceutical compound, the ratio of celecoxib to L-proline in step a) is 1:2.

One part of the co-crystal according to the invention, i.e. celecoxib, is a well-known drug with analgesic properties sometimes used for a long time worldwide. Due to this a further object of the present invention is a medicament comprising a co-crystal according to the invention.

Thus, the invention also concerns a medicament comprising at least one co-crystal according to the invention as described above and optionally one or more pharmaceutically acceptable excipients.

The invention also relates to a pharmaceutical composition that comprises a therapeutically effective amount of the co-crystal according to the invention in a physiologically acceptable medium.

The association of two active elements in the same pharmaceutical compound, such as a co-crystal, exhibits several advantages. Being linked, the elements often behave as a single chemical entity, thus facilitating the treatments, formulation, dosage etc. Another advantage is that the association of two active elements into one unique species seems to allow for a better Pharmacokinetic/Pharmacodynamic (PKPD) which helps in the treatment of pain.

In general in most embodiments in which the co-crystals of celecoxib and L-proline are used (e.g. for the treatment of pain) these compounds would be formulated into a convenient pharmaceutical formulation or a medicament. Accordingly, a desirable advantage of a pharmaceutical compound according to the present invention would show improved pharmaceutical properties and features, especially when compared to celecoxib alone and/or a mixture of celecoxib and L-proline. Thus, the pharmaceutical compound according to the invention should desirably show at least one, preferably more, of the following features:
● to have a very small particle size, e.g. from 300 µm or lower; or
● to be and/or remain essentially free of agglomerates; or
● to be less or not very hygroscopic; or
● to help in formulating controlled release or immediate release formulations; or
● to have a high chemical stability; or
   if given to a patient
● to decrease the inter- and intra-subject variability in blood levels; or
● to show a good absorption rate (e.g. increases in plasma levels or AUC); or
● to show a high maximum plasma concentration (e.g. Cₘₐₓ); or
● to show decreased time to peak drug concentrations in plasma (tₘₐₓ); or
● to show changes in half life of the compound (t_{½}), in whichever direction this change is preferably directed.

The medicament or pharmaceutical composition according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical composition of the present invention may for example be administered parenterally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments or pharmaceutical compositions according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) and Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical compositions according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

Typically, the medicaments or pharmaceutical compositions according to the present invention may contain 1-60 % by weight of one or more of the co-crystal and 40-99 % by weight of one or more auxiliary substances (additives/excipients).

The medicaments or pharmaceutical compositions of the present invention may also be administered topically or via a suppository.

The daily dosage for humans and animals may vary depending on factors that have their basis in the respective species or other factors, such as age, sex, weight or degree of illness and so forth. The daily dosage for humans of the co-crystal according to the present invention preferably is in the range of 1 to 2000 mg, preferably 5 to 500 milligrams being administered during one or several intakes per day.

In a further aspect the present invention relates to the use of a co-crystal according to the present invention for the treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder or dysmenorrhea. The use of the co-crystal according to the present invention might especially be drawn to the treatment of severe to moderate pain with an inflammatory component like e.g. rheumatoid arthritis, ankylosing spondylitis, sciatica and frozen shoulder. Preferably this use is provided in the form of a medicament or a pharmaceutical composition according to the invention as described above.

"Pain" is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

According to the IASP "allodynia" is defined as "a pain due to a stimulus which does not normally provoke pain" (IASP, Classification of chronic pain, 2nd Edition, IASP Press (2002), 210). Even though the symptoms of allodynia are most likely associated as symptoms of neuropathic pain this is not necessarily the case so that there are symptoms of allodynia not connected to neuropathic pain though rendering allodynia in some areas broader than neuropathic pain.

The IASP further draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response |
| | | mode differ |
| Hyperalgesia | Increased response | Stimulus and response |
| | | rate are the same |
| Hyperpathia | Raised threshold; | Stimulus and response |
| | Increased response | rate may be the same or |
| | | different |

According to the IASP "neuropathy" is defined as "a primary lesion or dysfunction in the nervous system" (IASP, Classification of chronic pain, 2nd Edition, IASP Press (2002), 211). Neuropathic pain may have central or peripheral origin.

"Sciatica" or "sciatic neuritis" is defined herein as a set of symptoms including pain that derive from irritation of the sciatic nerve or its roots.

"Frozen shoulder" or "adhesive capsulitis" is defined herein as a symptom wherein the connective tissue surrounding the shoulder joint or the shoulder capsule itself is causing chronic pain, becoming inflamed and stiff.

"Ankylosing spondylitis" or "Morbus Bechterew" is a chronic, inflammatory arthritis and autoimmune disease. It mainly affects joints in the spine and the sacroilium in the pelvis, causing eventual fusion of the spine.

"Dysmenorrhea" (or dysmenorrhoea) is a medical condition characterized by severe uterine pain during menstruation.

In another aspect the present invention relates to the use of a co-crystal according to the present invention for the treatment of familial adenomatons polyposis.

"Familial Adenomatous Polyposis" (FAP), also known as familial polyposis coli, is a hereditary disease characterised by the progressive appearance of numerous polyps mainly in the large intestine. Celecoxib is thought to induce cell death in FAP, and, therefore it is suggested that celecoxib prevents or delays the growth of polyps (http://www.emea.europa.eu/pdfs/human/comp/opinion/026404en.pdf).

A related further aspect of the invention is aimed at the use of the co-crystal according to the invention as described above in the manufacture of a medicament for the treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder, familial adenomatons polyposis or dysmenorrhea. This medicament might especially be drawn to the treatment of severe to moderate pain with an inflammatory component like e.g. rheumatoid arthritis, ankylosing spondylitis, sciatica and frozen shoulder.

Another aspect of the invention is related to the use of the co-crystal according to the invention as described above in the manufacture of a medicament for the treatment familial adenomatons polyposis.

One further aspect of the present invention is a method of treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder, familial adenomatons polyposis or dysmenorrhea, by providing to a patient in need thereof a sufficient amount of a co-crystal according to the invention as described above. This method of treatment might especially be relevant for the treatment of severe to moderate pain with an inflammatory component like e.g. rheumatoid arthritis, ankylosing spondylitis, sciatica and frozen shoulder. Preferably the co-crystal according to the invention is provided in physiologically suitable form like e.g. in the form of a medicament or a pharmaceutical composition according to the invention as described above.

Another aspect of the invention is a method of treatment of familial adenomatons by providing to a patient in need thereof a sufficient amount of a co-crystal according to the invention as described above. Preferably the co-crystal according to the invention is provided in physiologically suitable form like e.g. in the form of a medicament or a pharmaceutical composition according to the invention as described above.

The present invention is illustrated below with the help of the following figures and examples. These illustrations are given solely by way of example and do not limit the invention.

### Brief description of the figures:

**Figure 1** **:**
   Differential scanning calorimetry (DSC) analysis of the co-crystal celecoxib - L-proline (1:2).
**Figure 2****:**
   Thermogravimetric analysis (TGA) of the co-crystal celecoxib - L-proline (1:2).
**Figure 3****:**
   X-ray powder diffraction (XRPD) pattern of the co-crystal celecoxib - L-proline (1:2).
**Figure 4****:**
   Unit cell contents of the crystal structure of the co-crystal celecoxib ― L-proline (1:2); obtained by single crystal X-ray diffraction analysis (SCXRD) showing four molecules of L-proline and two molecules of celecoxib (hydrogen atoms have been omitted for clarity; program used: Mercury 2.2).

### EXAMPLES

### General Remarks:

Generally all figures of crystal structures are depicted with hydrogen atoms being omitted for clarity. The program used was Mercury 2.2.

In all XRPD measurements the 20 values were obtained using copper radiation (Cu_{kα} 1.54060 Å).

### Example: Celecoxib ― L-proline co-crystal (1:2)

### Process A (preparation by slurrying):

To a 10 mL flask equipped with magnetic stirrer containing celecoxib (509 mg, 1.34 mmol) and L-proline (307 mg, 2.68 mmol, 2 eq), was added 4 mL dichloromethane. The resultant suspension was stirred overnight. The solid was filtered with a sintered funnel (porosity 3) and washed with 1 mL cold dichloromethane. Traces of solvent were removed *in vacuo* at room temperature affording co-crystal celecoxib - L-proline (1:2) as a white powder (768 mg, 94%).

### Process B (preparation by crystallization):

To an assay tube equipped with magnetic stirrer containing celecoxib (69 mg, 0.18 mmol) and L-proline (42 mg, 0.36 mmol, 2 eq), was added the minimum amount of methanol to obtain complete dissolution at 64°C (vol. methanol 0.35 mL). The solution was allowed to cool to ambient temperature without stirring. Crystallization took place during the following 7 days. The product was filtered with a sintered funnel (porosity 3) and traces of solvent were removed *in vacuo* at room temperature affording co-crystal celecoxib - L-proline (1:2) as transparent rosettes (81 mg, 73%).

### CHARACTERISATION OF THE CO-CRYSTAL:

The celecoxib ― L-proline (1:2) co-crystal obtained according to the example was fully characterised by ¹H-NMR, FTIR, X-ray diffraction, Karl-Fisher analysis, DSC and TG and also single crystal X-ray diffraction analysis.

### ¹H NMR

Proton nuclear magnetic resonance analyses were recorded in methanol-d₄ in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR spectrum in methanol-d₄ at 400 MHz shows peaks at 7.99-7.90 (m, 2H); 7.53-7.45 (m, 2H); 7.23-7.13 (m, 4H); 6.90 (s, 1H); 3.96 (dd, J = 6.2 Hz, J = 8.6 Hz, 2H); 3.38 (dt, J = 7.0 Hz, J = 11.7 Hz, 2H); 3.22 (dt, J = 7.4 Hz, J = 11.7 Hz, 2H); 2.35 (s, 3H); 2.35-2.23 (m, 2H); 2.16-2.05 (m, 2H) 2.03-1.90 (m, 4H) ppm.

### IR

FTIR spectra were recorded using a Thermo Nicolet Nexus 870 FT-IR, equipped with a beamsplitter KBr system, a 35 mW He-Ne laser as the excitation source and a DTGS KBr detector. The spectra were acquired in 32 scans at a resolution of 4 cm⁻¹.

The sample (KBr pellet) shows a Fourier Transform Infra Red spectrum with absorption bands at 3283.4 (m), 3079.5 (m), 2985.0 (m), 2680.0 (w), 1599.0 (m), 1500.1 (m), 1469.8 (s), 1449.2 (m), 1373.7 (s), 1345.4 (s), 1331.6 (s), 1267.3 (m), 1231.8 (s), 1172.0 (s), 1137.1 (s), 1111.9 (m), 1093.9 (m), 975.5 (m), 841.3 (s), 817.0 (s), 760.5 (m), 684.0 (m), 627.0 (s), 616.4 (s) 557.3 (m), 544.5 (m), 479.1 (m) cm⁻¹.

### DSC

Differential scanning calorimetry (DSC) analyses were recorded with a Mettler DSC822^{e}. A sample of 5.0140 mg was weighed into 40 µL aluminium crucible with a pinhole lid and was heated, under nitrogen (50 mL/min), at 10°C/min from 30 to 300°C.

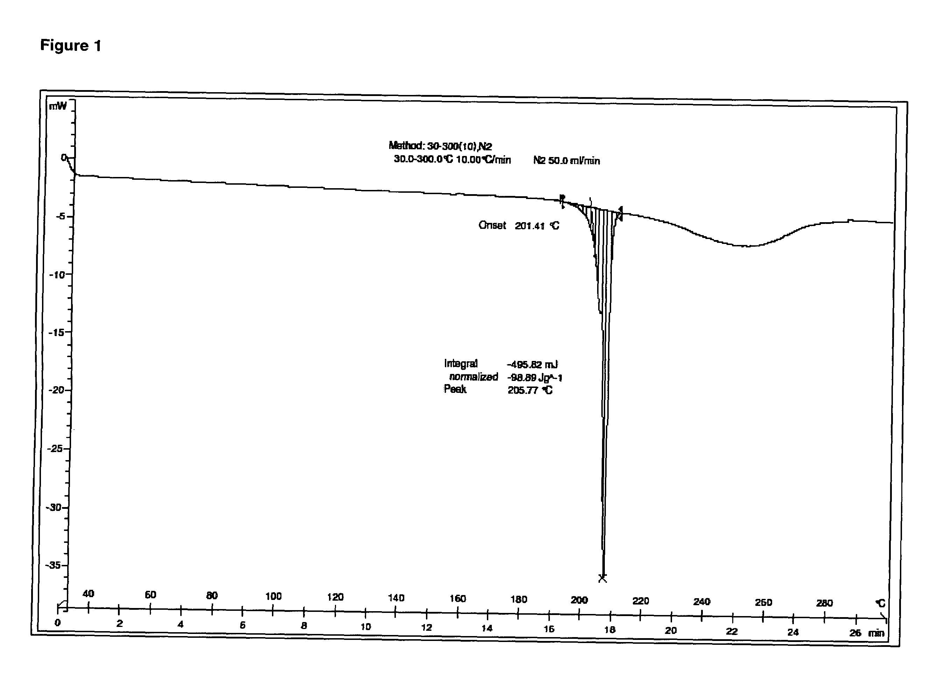

The novel type of crystal of the present invention is characterized in that the endothermic sharp peak corresponding to the melting point has an onset at 201.4°C (fusion enthalpy - 98.89 J/g), measured by DSC analysis (10 °C/min) (see figure 1).

### TGA

Thermogravimetric analyses (TGA) were recorded in a thermogravimetric analyzer Mettler TGA/SDTA851^{e}. A sample of 4.5714 mg was weighed into a 70 µL alumina crucible with a pinhole lid and was heated at 10 °C/min from 30 to 300°C, under nitrogen (50 mL/min).

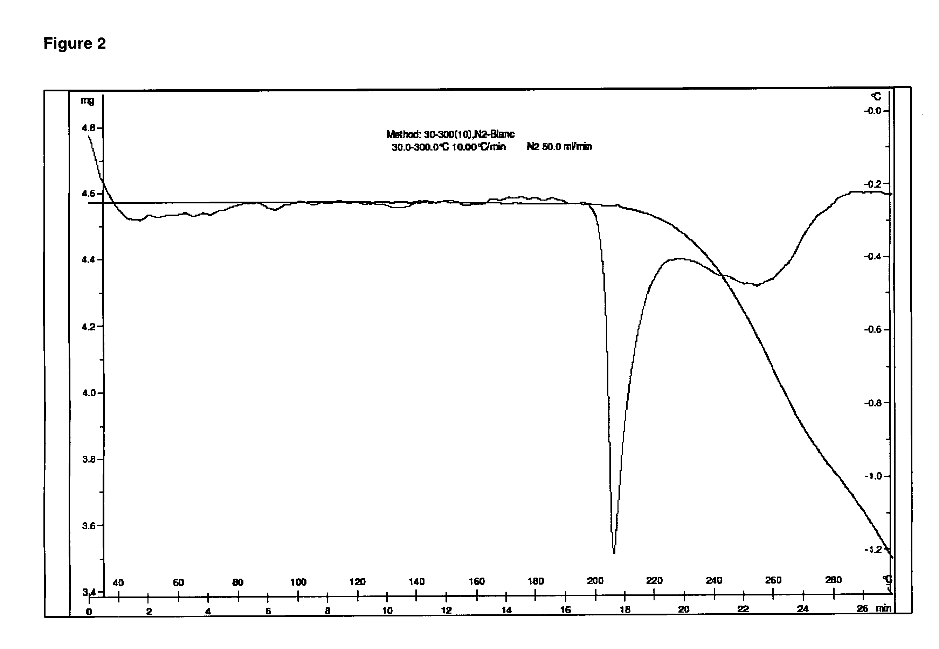

The TG analysis of the crystalline form according to the invention shows no weight loss between 30°C and its melting point (see figure 2).

### XRPD

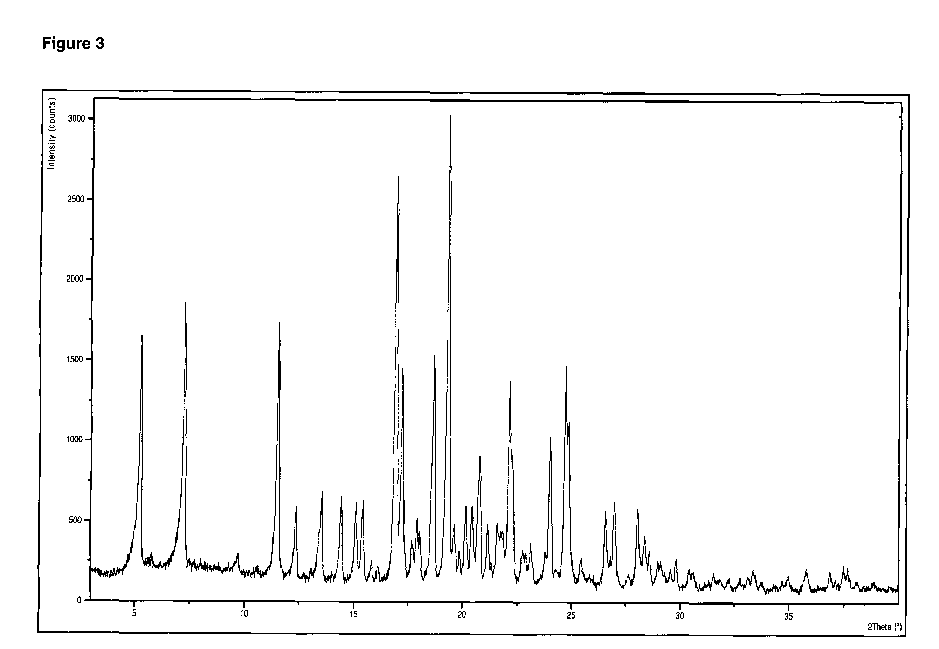

X-ray powder diffraction (XRPD) analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 8.8° per minute (see figure 3).

**List of selected peaks:**

| **2θ(_{°})** | **d(Å)** | **l(%)** |
|---|---|---|
| 5.29 | 16.72 | 54 |
| 5.77 | 15.32 | 2 |
| 7.28 | 12.14 | 59 |
| 9.71 | 9.11 | 4 |
| 10.60 | 8.35 | 1 |
| 11.57 | 7.65 | 58 |
| 12.39 | 7.15 | 15 |
| 13.05 | 6.79 | 1 |
| 13.56 | 6.53 | 19 |
| 14.46 | 6.13 | 19 |
| 15.11 | 5.86 | 17 |
| 15.45 | 5.74 | 19 |
| 15.83 | 5.60 | 4 |
| 16.14 | 5.49 | 3 |
| 16.96 | 5.23 | 94 |
| 17.25 | 5.14 | 48 |
| 17.67 | 5.02 | 7 |
| 17.92 | 4.95 | 13 |
| 18.08 | 4.91 | 8 |
| 18.70 | 4.74 | 48 |
| 19.37 | 4.58 | 100 |
| 19.64 | 4.52 | 10 |
| 19.85 | 4.47 | 4 |
| 20.17 | 4.40 | 15 |
| 20.43 | 4.35 | 15 |
| 20.79 | 4.27 | 27 |
| 21.17 | 4.20 | 10 |
| 21.58 | 4.12 | 11 |
| 21.85 | 4.07 | 10 |
| 22.15 | 4.01 | 47 |
| 22.30 | 3.99 | 26 |
| 22.74 | 3.91 | 6 |
| 23.12 | 3.85 | 9 |
| 23.78 | 3.74 | 6 |
| 24.04 | 3.70 | 34 |
| 24.72 | 3.60 | 51 |
| 24.87 | 3.58 | 37 |
| 25.43 | 3.50 | 5 |
| 26.57 | 3.35 | 17 |
| 26.97 | 3.31 | 19 |
| 27.66 | 3.23 | 2 |
| 28.04 | 3.18 | 17 |
| 28.37 | 3.15 | 11 |
| 28.59 | 3.12 | 7 |
| 29.11 | 3.07 | 5 |
| 29.56 | 3.02 | 3 |
| 29.78 | 3.00 | 5 |
| 30.41 | 2.94 | 3 |
| 30.60 | 2.92 | 3 |
| 31.56 | 2.83 | 3 |
| 32.21 | 2.78 | 1 |
| 32.74 | 2.74 | 2 |
| 33.13 | 2.70 | 3 |
| 33.35 | 2.69 | 4 |
| 33.75 | 2.66 | 1 |
| 34.97 | 2.57 | 3 |
| 35.80 | 2.51 | 4 |
| 36.87 | 2.44 | 3 |
| 37.48 | 2.40 | 5 |
| 37.69 | 2.39 | 4 |
| 38.08 | 2.36 | 1 |
| 38.87 | 2.32 | 1 |

### Single crystal X-ray diffraction

Crystal structure of co-crystal celecoxib - L-proline (1:2) has been determined from single crystal X-ray diffraction data. The colourless prismatic crystal used (0.52 x 0.33 x 0.33 mm) was obtained from the crystallization of a solution in toluene and methanol of equimolar amounts of celecoxib and L-proline.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ²) against all measured intensities was carried out (program used: SHELXTL-NT 6.1). All non-hydrogen atoms were refined with anisotropic displacement parameters. Fluorine atoms and some carbons in the structure were found to be affected by disorder.

**Relevant structural data:**

| Crystal system | Orthorhombic |
|---|---|
| Space group | *P*2₁2₁2₁ |
| a (Å) | 9.6086(4) |
| b (Å) | 20.0675(7) |
| c (Å) | 30.6272(12) |
| Volume (Å³) | 5905.6(4) |
| Z | 8 |
| D calc. (Mg/m³) | 1.38 |
| N.of refl. | 14575 |
| Refl. with l >2σ(l) | 10837 |
| R (l > 2σ(l)) | 0.0614 |

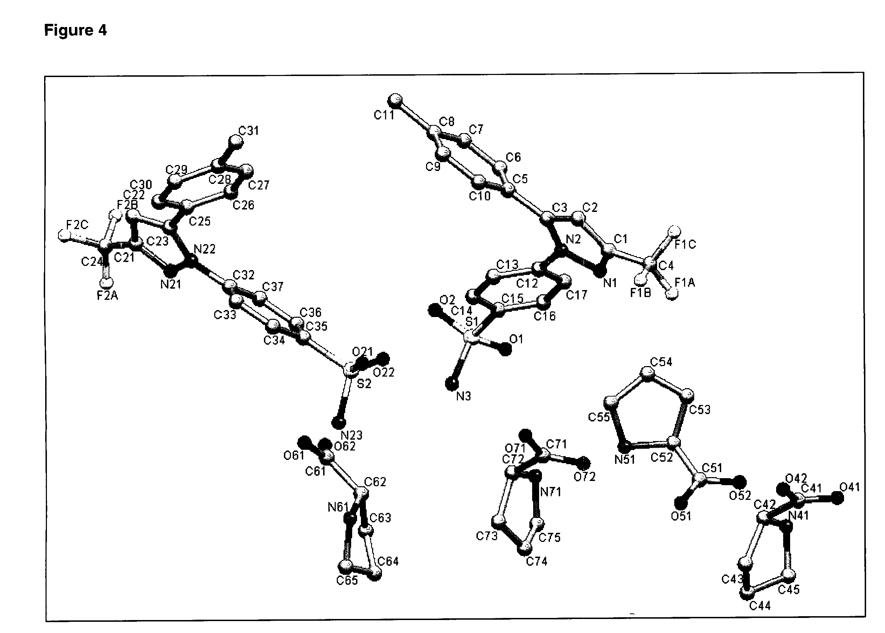

The asymmetric unit of the crystal structure of co-crystal celecoxib ― L-proline (1:2) is depicted in figure 4 (hydrogen atoms have been omitted for clarity; program used: *Mercury* 2.2).

Simulation of XRPD diffractogram from single crystal data gives an almost identical diagram to the experimental one presented above.

## Claims

1. Co-crystal comprising celecoxib either as a free base or as a physiologically salt and L-proline either as a free base or as a physiologically salt.

2. Co-crystal according to claim 1, wherein the ratio between celecoxib and L-proline is chosen in such a way that if compared to either celecoxib alone and/or to a mixture of celecoxib and L-proline
● the solubility of the co-crystal is increased; and/or
● the dose response of the co-crystal is increased; and/or
● the efficacy of the co-crystal is increased; and/or
● the dissolution of the co-crystal is increased; and/or
● the bioavailability of the co-crystal is increased; and/or
● the stability of the co-crystal is increased; and/or
● the hygroscopicity of the co-crystal is decreased; and/or
● the form diversity of the co-crystal is decreased; and/or
● the morphology of the co-crystal is modulated.

3. Co-crystal according to claim 1 or 2, wherein the molecular ratio between celecoxib and L-proline is 1:2.

4. Co-crystal according to any of claims 1 to 3, **characterized in that** the endothermic sharp peak corresponding to the melting point has an onset at 201.4°C.

5. Co-crystal according to any of claims 1 to 4, **characterized in that** it shows a X-Ray powder diffraction pattern with peaks [2θ] at 5.29, 5.77, 7.28, 9.71, 10.60, 11.57, 12.39, 13.05, 13.56, 14.46, 15.11, 15.45, 15.83, 16.14, 16.96, 17.25, 17.67, 17.92, 18.08, 18.70, 19.37, 19.64, 19.85, 20.17, 20.43, 20.79, 21.17, 21.58, 21.85, 22.15, 22.30, 22.74, 23.12, 23.78, 24.04, 24.72, 24.87, 25.43, 26.57, 26.97, 27.66, 28.04, 28.37, 28.59, 29.11, 29.56, 29.78, 30.41, 30.60, 31.56, 32.21, 32.74, 33.13, 33.35, 33.75, 34.97, 35.80, 36.87, 37.48, 37.69, 38.08 and 38.87.

6. Co-crystal according to any of claims 1 to 5, **characterized in that** it has a orthorhombic unit cell with the following dimensions:
a = 9.61 Å,
b = 20.07 Å, and
c = 30.63 Å.

7. Process for the production of a co-crystal according to any of claims 1 to 6 comprising the steps of:
(f) dissolving or suspending celecoxib and L-proline in a solvent or mixture of solvents,
(g) optionally mixing or stirring the solution or suspension,
(h) optionally before, during or after step (b) cooling or keeping the mixed solution/suspension to or at ambient temperature or below;
(i) optionally filtering-off and/or washing the resulting solid with a solvent, and
(j) drying the solid optionally at ambient temperature and/or vacuum.

8. Medicament comprising at least one co-crystal according to any of claims 1 to 6 and optionally one or more pharmaceutically acceptable excipients.

9. Pharmaceutical composition **characterized in that** it comprises a therapeutically effective amount of the co-crystal according to any of claims 1 to 6 in a physiologically acceptable medium.

10. Use of a co-crystal according to any one of claims 1 to 6 for the treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder or dysmenorrhea.

11. Use of a co-crystal according to any one of claims 1 to 6 for the treatment of familial adenomatons polyposis.
